(19) Europäisches Patentamt
European Patent Office
Office européen des brevets

(11) **EP 4 205 550 A1**

(12) **EUROPEAN PATENT APPLICATION**
published in accordance with Art. 153(4) EPC

(43) Date of publication:
**05.07.2023 Bulletin 2023/27**

(21) Application number: **21861726.4**

(22) Date of filing: **27.08.2021**

(51) International Patent Classification (IPC):
***A23F 5/24*** *(2006.01)*

(52) Cooperative Patent Classification (CPC):
**A23F 5/24**

(86) International application number:
**PCT/JP2021/031574**

(87) International publication number:
**WO 2022/045305 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2020 JP 2020145700**

(71) Applicants:
• **Amano Enzyme Inc.
Nagoya-shi
Aichi 460-8630 (JP)**

• **Amano Enzyme U.S.A. Co., Ltd.
Elgin, Illinois 60124 (US)**

(72) Inventors:
• **OKUDA, Keita
Elgin, Illinois 60124 (US)**
• **LIOUTAS, Theodore
Oak Brook, Illinois 60523 (US)**

(74) Representative: **Müller-Boré & Partner
Patentanwälte PartG mbB
Friedenheimer Brücke 21
80639 München (DE)**

(54) **COFFEE EXTRACT PRODUCTION METHOD AND ENZYME PREPARATION**

(57)     The purpose of the present invention is to provide a technique for producing a coffee extract, the technique being capable of further reducing the turbidity of the coffee extract. Provided are: a method which is for producing a coffee extract with reduced turbidity and includes a step for bringing a coffee extract into contact with glucoamylase of which the glucoamylase activity per 1 g of coffee beans is 32 U or less; and a method which is for producing a coffee extract with reduced turbidity and includes a step for bringing a coffee extract into contact with glucoamylase and galactomannanase, wherein the glucoamylase is used such that the glucoamylase activity is 0.24 U or more per 1 U of the galactomannanase activity.

**Description**

TECHNICAL FIELD

The present invention relates to a method for producing a coffee extract and an enzyme preparation. More specifically, the present invention relates to a method for producing a coffee extract having reduced turbidity, and an enzyme preparation for use of reducing the turbidity of a coffee extract.

BACKGROUND ART

The turbidity of the coffee extract causes a decrease in production efficiency due to adhesion of insoluble matter to the device or the like in the production process, and causes a decrease in commercial value due to deterioration of tongue feeling and flavor or the like in the storage process.

In order to prevent the generation of the turbidity of a coffee extract, a method for enzymatically treating the coffee extract is known. For example, a method in which a fibrous degrading enzyme such as pectinase, cellulase, hemicellulase, arabanase, or β-glucanase is allowed to act on a coffee extract before a sterilization step to prevent the turbidity of the coffee extract (Patent Document 1); a method for producing a coffee beverage including a step of treating a raw material component of a coffee beverage containing a coffee extract or a coffee eluate with an enzyme derived from a filamentous fungus (*Aspergillus niger*) having galactomannanase activity and acid protease activity (Patent Document 2); a method for producing concentrated coffee, including preparing a concentrated coffee liquid containing 5 to 35 wt% of a solid content, and then adding a galactomannan degrading enzyme to the concentrated coffee liquid (Patent Document 3); and a method for producing a coffee beverage including a step of treating a coffee liquid with a galactomannan degrading enzyme (Patent Document 4), have been proposed.

PRIOR ART DOCUMENTS

PATENT DOCUMENTS

Patent Document 1: Japanese Patent Laid-open Publication No. H04-045745
Patent Document 2: Japanese Patent Laid-open Publication No. 2002-272375
Patent Document 3: Japanese Patent Laid-open Publication No. 2002-330700
Patent Document 4: Japanese Patent Laid-open Publication No. 2003-047406

SUMMARY OF THE INVENTION

PROBLEMS TO BE SOLVED BY THE INVENTION

In the conventional method for producing a coffee extract using an enzyme treatment, it cannot be said that turbidity can be sufficiently reduced.

The purpose of the present invention is to provide a technique for producing a coffee extract, the technique being capable of further reducing the turbidity of the coffee extract.

MEANS FOR SOLVING THE PROBLEM

The present inventor has found that glucoamylase is extremely excellent in an effect of reducing the turbidity of a coffee extract, and based on this, it has been found that the turbidity can be effectively reduced by using a relatively small amount of glucoamylase, and the turbidity can be remarkably reduced by using a combination of glucoamylase at a predetermined ratio or more to galactomannanase. That is, the present invention provides inventions of the following aspects.

Item 1. A method for producing a coffee extract, the method comprising a step of bringing a coffee extract into contact with glucoamylase having a glucoamylase activity of 32 U or less per 1 g of coffee beans.

Item 2. The method according to item 1, in which in the step, a coffee extract is extracted from a slurry containing ground coffee beans, water, and the glucoamylase.

Item 3. The method according to item 1 or 2, in which the glucoamylase is used at a glucoamylase activity of 0.5 U or more per 1 g of the coffee beans.

Item 4. A method for producing a coffee extract, the method comprising a step of bringing a coffee extract into contact with glucoamylase and galactomannanase, in which the glucoamylase is used at a glucoamylase activity of 0.24 U or more per 1 U of the galactomannanase activity.

Item 5. The method according to item 4, in which in the step, a coffee extract is extracted from a slurry containing ground coffee beans, water, the glucoamylase, and the galactomannanase.

Item 6. The method according to item 4 or 5, in which the glucoamylase is used at a glucoamylase activity of 2 U or less per 1 U of the galactomannanase activity.

Item 7. The method according to any one of items 1 to 6, in which the glucoamylase is used at a glucoamylase activity of 20 U or less per 1 g of coffee beans. Item 8. The method according to any one of items 1 to 7, in which the glucoamylase is derived from Rhizopus oryzae.

Item 9. An enzyme preparation containing glucoamylase for the use of reducing turbidity of a coffee extract.

Item 10. The enzyme preparation according to item 9, for the use in an amount that the glucoamylase activity is 32 U or less per 1 g of coffee beans.

Item 11. The enzyme preparation according to item 9 or 10, further comprising galactomannanase, in which a content of the glucoamylase per 1 U of the galactomannanase activity is 0.24 U of glucoamylase activity.

ADVANTAGES OF THE INVENTION

According to the present invention, there is provided a technique for producing a coffee extract capable of further reducing the turbidity of the coffee extract.

BRIEF DESCRIPTION OF THE DRAWINGS

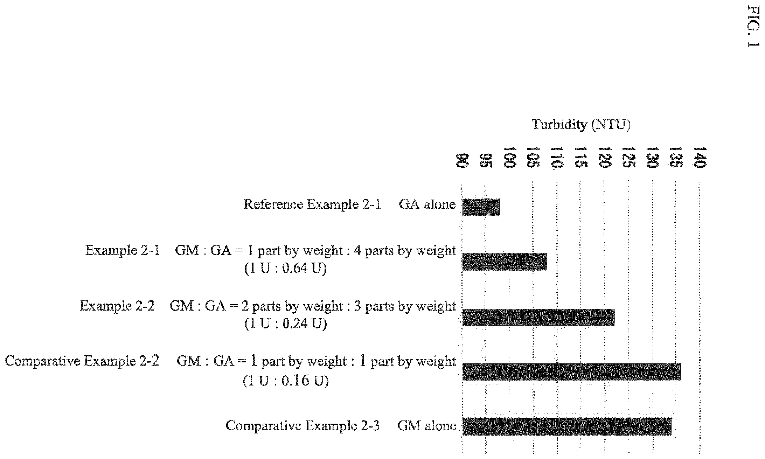

Fig. 1 is a graph comparing the turbidity (NTU) of coffee extracts obtained in Reference Example 2-1 (when only glucoamylase is used), Examples 2-1 to 2-2 (when glucoamylase and galactomannanase are used in combination at a predetermined ratio), Comparative Example 2-2 (when glucoamylase and galactomannanase are used in combination at a ratio deviating from the predetermined ratio), and Comparative Example 2-3 (when only galactomannanase is used).

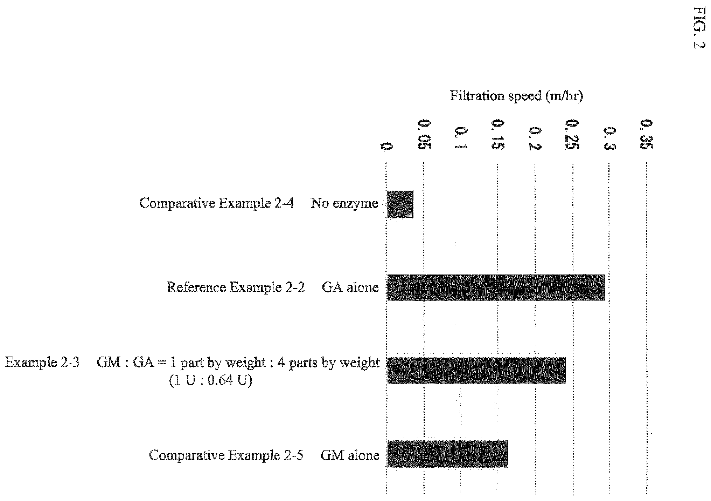

Fig. 2 is a graph comparing the filtration speeds (m/h) of coffee extracts obtained in Comparative Example 2-4 (when no enzyme was used), Reference Example 2-2 (when only glucoamylase was used), Example 2-3 (when glucoamylase and galactomannanase were used in combination at a predetermined ratio), and Comparative Example 2-5 (when only galactomannanase was used).

EMBODIMENTS OF THE INVENTION

1. Method for producing coffee extract

1-1. Enzyme treatment step

A first embodiment of the method for producing a coffee extract of the present invention includes a step (enzyme treatment step) of bringing a coffee extract into contact with glucoamylase having a glucoamylase activity of 32 U or less per 1 g of coffee beans, to obtain a coffee extract.

Since glucoamylase is extremely excellent in an effect of reducing the turbidity of a coffee extract, the effect of reducing the turbidity of a coffee extract can be effectively obtained even when the amount of glucoamylase used per predetermined amount of coffee beans is small. In addition, glucoamylase is extremely excellent in an effect of improving the filtration speed of a coffee extract in addition to the effect of reducing the turbidity of a coffee extract, therefore, even when the amount of glucoamylase used per predetermined amount of coffee beans is small, the effect of improving the filtration speed of a coffee extract can be effectively obtained. Therefore, from the viewpoint of the balance between the turbidity reduction effect (or the turbidity reduction effect and the filtration speed improvement effect) of a coffee extract and the saving of glucoamylase, preferred examples of the amount of glucoamylase used in the first embodiment of the present invention include a glucoamylase activity of preferably 16 U or less, more preferably 14 U or less, further preferably 12 U or less, still more preferably 10 U or less, further still more preferably 8 U or less, and particularly preferably 7 U or less.

In the first embodiment of the method for producing a coffee extract of the present invention, the lower limit of the range of the amount of glucoamylase used per 1 g of coffee beans is not particularly limited, and may be appropriately determined according to the degree to which the turbidity in the coffee extract should be reduced (or the degree to which the turbidity should be reduced and the degree to which the filtration speed should be improved), but the lower limit is preferably 1 U or more of glucoamylase activity per 1 g of coffee beans. From the viewpoint of further enhancing the coffee extract turbidity reducing effect (or the turbidity reducing effect and the filtration speed improving effect), the amount of glucoamylase used per 1 g of coffee beans is preferably 0.5 U or more, more preferably 0.8 U or more, further preferably 1 U or more, still more preferably 1.3 U or more, and particularly preferably 1.5 U or more.

In the present invention, regarding the activity value of glucoamylase, the amount of enzyme that increases the reducing power corresponding to 1 mg of glucose per minute is defined as 1 U.

A second embodiment of the method for producing a coffee extract of the present invention includes a step (enzyme treatment step) of bringing a coffee extract into contact with glucoamylase and galactomannanase, wherein the glucoamylase is used at a glucoamylase activity of 0.24 U or more per 1 U of the galactomannanase activity, to obtain a coffee extract.

Glucoamylase can increase the soluble solid content in a coffee extract by combining galactomannanase. On the other hand, galactomannanase not only has a poor effect of reducing the turbidity of a coffee extract, but also partially loses the effect of reducing the turbidity of a coffee extract by glucoamylase when combined with glucoamylase. Furthermore, galactomannanase not only has a poor effect of improving the filtration speed of a coffee extract, but also partially loses the effect of improving the filtration speed of a coffee extract by glucoamylase when combined with glucoamylase. However, since glucoamylase is extremely excellent in the coffee extract turbidity reducing effect (or the turbidity reducing effect and the filtration speed improving effect), in the second embodiment of the method for producing a coffee extract of the present invention, by using glucoamylase such that the glucoamylase activity is 0.24 U or more per 1 U of galactomannanase activity, it is possible to suppress the loss of the coffee extract turbidity reducing effect (or the turbidity reducing effect and the filtration speed improving effect) due to the combination with glucoamylase, and to effectively obtain the coffee extract turbidity reducing effect (or the turbidity reducing effect and the filtration speed improving effect).

In the second embodiment of the method for producing a coffee extract of the present invention, the amount of glucoamylase used per 1 U of galactomannanase activity is preferably a glucoamylase activity of 0.4 U or more, more preferably 0.5 U or more, and still more preferably 0.6 U or more, from the viewpoint of further enhancing the coffee extract turbidity reducing effect (or the turbidity reducing effect and the filtration speed improving effect).

In the second embodiment of the method for producing a coffee extract of the present invention, the upper limit of the range of the use amount of glucoamylase per 1 U of galactomannanase activity is not particularly limited, and examples thereof include a glucoamylase activity of 2 U or less. Since glucoamylase is extremely excellent in a coffee extract turbidity reducing effect (or the turbidity reducing effect and the filtration speed improving effect), it is possible to effectively obtain the coffee extract turbidity reducing effect (or the turbidity reducing effect and the filtration speed improving effect) without using a large amount of glucoamylase per galactomannanase activity. From the viewpoint of saving glucoamylase, the upper limit of the range of the amount of glucoamylase used per 1 U of galactomannanase activity is preferably a glucoamylase activity of 1.5 U or less, more preferably 1.3 U or less, further preferably 1 U or less, still more preferably 0.8 U or less, and particularly preferably 0.7 U or less.

In the present invention, regarding the activity value of galactomannanase, the amount of enzyme that leads to an increase in the reducing power corresponding to 1 $\mu$mol of mannose per minute is defined as 1 U.

In the second embodiment of the method for producing a coffee extract of the present invention, the amount of glucoamylase used per coffee beans is not particularly limited, but the glucoamylase can be used in the amount described in the first embodiment.

That is, since glucoamylase is extremely excellent in an effect of reducing the turbidity of a coffee extract, and can effectively obtain the effect of reducing the turbidity of a coffee extract (or the turbidity reducing effect and the filtration speed improving effect) even when the amount of glucoamylase used per a predetermined amount of coffee beans is relatively small, from the viewpoint of the balance between the effect of reducing the turbidity of a coffee extract (or the turbidity reducing effect and the filtration speed improving effect) and saving of glucoamylase, the suitable example of the amount of glucoamylase used in the second embodiment of the present invention is, for example, a glucoamylase activity of 32 U or less, preferably 16 U or less, more preferably 14 U or less, further preferably 12 U or less, still more preferably 10 U or less, further still more preferably 8 U or less, and particularly preferably 7 U or less.

In the second embodiment of the method for producing a coffee extract of the present invention, the lower limit of the range of the amount of glucoamylase used per 1 g of coffee beans is not particularly limited, and may be appropriately determined according to the degree to which the turbidity in the coffee extract should be reduced (or the degree to which the turbidity should be reduced and the degree to which the filtration speed should be improved), and the glucoamylase activity is, for example, 1 U or more per 1 g of coffee beans, and the glucoamylase activity is preferably 1.5 U or more, more preferably 2 U or more, further preferably 4 U or more, still more preferably 4.5 U or more, further still more preferably 5 U or more, and particularly preferably 6 U or more from the viewpoint of further enhancing the coffee extract turbidity reduction effect (or the turbidity reduction effect and the filtration speed improvement effect).

The coffee beans used in the present invention are not particularly limited in both the first embodiment and the second embodiment as long as they are roasting coffee beans suitable for coffee extraction. Therefore, the production area of coffee beans is not particularly limited, and examples thereof include robusta type (Indonesia, Vietnam, Uganda) and arabica type (Brazil, Kilimanjaro, Peru, Columbia, Guatemala), and these coffee beans can be used alone or in combination of two or more types. In addition, the roasting level of the coffee beans is not particularly limited, and examples thereof include light roasting, cinnamon roasting, medium roasting, high roasting, city roasting, full city roasting, French roasting, and Italian roasting, and beans having these roasting levels can be used alone or in combination of two or more kinds having different roasting levels.

In the present invention, in the step of bringing a coffee extract into contact with the enzyme (glucoamylase, or glucoamylase and galactomannanase) (enzyme treatment step), the timing of bringing the coffee extract into contact with the enzyme is not particularly limited in both the first embodiment and the second embodiment. For example, a coffee extract (coffee extraction liquid) may be prepared in advance by extracting ground coffee beans, and then the coffee extract and the enzyme may be mixed, or the coffee extract may be extracted from a slurry containing the ground coffee beans, water, and the enzyme, thereby preparing the coffee extract and bringing the coffee extract and the enzyme into contact with each other at substantially the same time. In the present invention, it is preferable to simultaneously perform the preparation of the coffee extract and the contact between the coffee extract and the enzyme. The water contained in the slurry refers to water whose temperature is not limited and includes non-heated water and heated water (hot water or the like). The slurry may also contain a coffee extract, wherein the slurry can contain ground coffee beans, a coffee extract (a liquid containing a water extract of coffee in water), and an enzyme.

In the present invention, the pH condition, and the temperature condition at the time of bringing the coffee extract and the enzyme (glucoamylase, or glucoamylase and galactomannanase) into contact with each other can be appropriately determined according to the optimum pH and the optimum temperature of the enzyme to be used. For example, the pH condition is, for example, 3 to 8, preferably 4 to 7, further preferably 5 to 6, still more preferably 5 to 5.5, and the temperature condition is, for example, 10 to 50°C, preferably 20 to 40°C.

The temperature condition and the pressure condition in the extraction of coffee are not particularly limited. The temperature condition may be either water extraction (non-warming extraction) or heating extraction, but water extraction is preferable. In addition, when the preparation of the coffee extract and the contact of the coffee extract with the enzyme are simultaneously performed, the temperature condition may be determined according to the optimal temperature of the enzyme, and in this case, the temperature condition is specifically, for example, 10 to 50°C, preferably 20 to 40°C. The pressure condition may be either pressurized extraction or non-pressurized extraction.

In the present invention, the time for bringing the coffee extract into contact with the enzyme (glucoamylase, or glucoamylase and galactomannanase) is not particularly limited, and is, for example, 30 minutes to 5 hours, preferably 1 to 4 hours, further preferably 1.5 to 3 hours.

The glucoamylase used in the present invention is an enzyme having exo-1,4-$\alpha$-glucosidase activity (EC 3.2.1.3), and specific examples in both the first embodiment and the second embodiment preferably include glucoamylase derived from the genus Aspergillus, such as Rhizopus, Eudomyces, Penicillium, Nurospora, Trichoderma, and Mucor. The glucoamylase derived from the genus Rhizopus is not particularly limited, and examples thereof include Rhizopus oryzae, Rhizopus delemer, and Rhizopus niveus. These glucoamylases may be used singly or in combination of two or more kinds thereof.

Among these glucoamylase, glucoamylase derived from the genus Rhizopus is further preferable, and glucoamylase derived from Rhizopus oryzae is still more preferable, from the viewpoint of further enhancing the coffee extract turbidity reducing effect (or the turbidity reducing effect and the filtration speed improving effect).

The titer of glucoamylase is, for example, 500 U/g or more, preferably 1000 U/g or more, and more preferably 1500 U/g or more. The upper limit of the range of the titer of glucoamylase is not particularly limited, and is, for example, 10,000 U/g or less, preferably 5000 U/g or less, and more preferably 2000 U/g or less.

The galactomannanase used in the second embodiment of the method for producing a coffee extract of the present invention is an enzyme having endo-1,4-$\beta$-mannanase activity (EC 3.2.1.78), and specific examples thereof include, but are not particularly limited to, galactomannanase derived from the genus Aspergillus. The galactomannanase derived from the genus Aspergillus is preferably a galactomannanase derived from Aspergillus niger.

The titer of galactomannanase is, for example, 1000 U/g or more, preferably 5000 U/g or more, and more preferably 8000 U/g or more. The upper limit of the range of the titer of galactomannanase is not particularly limited, but it is, for example, 50,000 U/g or less, preferably 30,000 U/g or less, and more preferably 15000 U/g or less.

1-2. Other steps

In the present invention, the coffee extract having reduced turbidity (or reduced turbidity and improved filtration speed) obtained by the enzyme treatment step is appropriately subjected to the enzyme deactivation step in both the first embodiment and the second embodiment; coffee bean separation step by centrifugation, stationary separation, filtration, or the like; and/or a sterilization step. The order of the enzyme deactivation step, the coffee bean separation step and the sterilization step is arbitrary.

1-3. Use of coffee extract

The coffee extract with reduced turbidity (or with reduced turbidity and improved filtration speed) obtained by the present invention may be used as it is for production of coffee foods and beverages or may be prepared as a concentrated liquid or a dried extract by appropriately removing moisture, and then diluted by adding water as necessary, and used for production of coffee foods and beverages.

Among coffee foods and beverages, the coffee beverages include, for example, sugar-free black coffee; sweetened black coffee to which sucrose, liquid sugar, sweetener, and the like are added; and a cafe-au-lait-type coffee beverage in which milk components such as milk, nonfat dry milk, and fresh cream are added to a sugarless or sugar-added coffee beverage.

Among coffee foods and beverages, the coffee foods include, for example, coffee-flavored frozen confectionery/chilled confectionery such as jelly, pudding, ice cream, and ice candy; coffee-flavored confectionary/bakery products such as cakes, candies, cookies, and breads.

In the production of coffee foods and beverages, any material that can be used for coffee foods and beverages can be added to the coffee extract having reduced turbidity (or reduced turbidity and improved filtration speed). Examples of the optional material include milk components, saccharides, sweeteners, salt, flour, eggs, and the like. Furthermore, in the production of coffee foods and beverages, any component that can be used for coffee foods and beverages can be added to the coffee extract having a reduced turbidity (or having a reduced turbidity and an improved filtration speed). Examples of the optional component include an antioxidant, a pH adjusting agent, an emulsifier, a fragrance, a stabilizer, an antioxidant, a preservative, and the like.

2. Enzyme preparation for use of reducing turbidity of coffee extract

As described above, in both the first embodiment and the second embodiment in the production method of the present invention, glucoamylase can remarkably reduce the turbidity of a coffee extract. Therefore, the present invention further provides an enzyme preparation containing glucoamylase for use of reducing the turbidity of a coffee extract. As described above, glucoamylase can remarkably improve the filtration speed of a coffee extract. Therefore, the enzyme preparation of the present invention can also be used for improving the filtration speed.

As shown in the first embodiment and the second embodiment in the production method of the present invention, the enzyme preparation of the present invention can be used in an amount of 32 U or less per 1 g of coffee beans. In addition, as shown in the second embodiment in the production method of the present invention, the enzyme preparation of the present invention may further contain galactomannanase, and the content of the glucoamylase per 1 U of the galactomannanase activity may be 0.24 U.

Reducing the turbidity of a coffee extract means obtaining a coffee extract having a turbidity lower than the turbidity of a coffee extract obtained when the enzyme treatment is not performed. The degree of reduction in the turbidity of a coffee extract is preferably 0.4 or less, more preferably 0.3 or less as the relative turbidity when the turbidity of a coffee extract obtained when the enzyme treatment is not performed is 1. The turbidity can be determined, for example, as the turbidity (NTU) when the coffee extract is diluted with water so that Brix = 2.

In addition, improving the filtration speed of a coffee extract means obtaining a coffee extract having a filtration speed higher than the filtration speed of a coffee extract obtained when the enzyme treatment is not performed. The degree of improvement in the filtration speed of the coffee extract is preferably 5.5 or more, more preferably 6 or more, and still more preferably 6.5 or more, as the relative filtration speed when the filtration speed of a coffee extract obtained without enzyme treatment is 1. The filtration speed can be determined as a value based on the amount of filtrate ($m^3$) $\times$ 1/filtration area ($m^2$) $\times$ 1/hour (hr).

In the enzyme preparation of the present invention, the details of each enzyme, the method of use, and the like are as described above in "1. Method for producing coffee extract".

In addition to glucoamylase and galactomannanase, the enzyme preparation of the present invention may contain other components that are sitologically acceptable. Examples of the other components include excipients, disintegrants, preservatives, preservatives, stabilizers, vitamins, minerals, sweeteners, seasonings, and the like.

EXAMPLES

Hereinafter, the present invention will be specifically described with reference to Examples, but the present invention is not to be construed as being limited to the following Examples.

(1) Activity measurement method

The method for measuring the titer (activity value) of glucoamylase and galactomannanase is as follows.

<Glucoamylase (GA) activity>

The glucoamylase activity was measured by the following method according to ninth edition of the Japan's Specifications and Standards of Food Additives, Glucoamylase Activity Test Method, Method 4.

Here, 0.50 g of an enzyme sample was weighed, and diluted to an appropriate concentration by adding water, and this was used as a sample solution. Potato starch was previously dried at 105°C for 2 hours, 1.0 g of the dried product was weighed, 20 mL of water was added thereto, and 5 mL of a sodium hydroxide test solution (2 mol/L) was gradually added thereto while stirring to form a paste. Gelatinous starch was heated in a water bath for 3 minutes with stirring, then 25 mL of water was added thereto, and the mixture was cooled, then neutralized by adding a hydrochloric acid test solution (2 mol/L) and a hydrochloric acid test solution (0.1 mol/L), 10 mL of a 1 mol/L acetic acid-sodium acetate buffer solution (pH 4.5) was added thereto, and water was further added thereto to make 100 mL, thereby obtaining a substrate solution.

Here, 10 mL of the substrate solution was weighed, and warmed at 37°C for 10 minutes, 1 mL of the sample solution was added, and immediately shaken, and warmed at 37°C for 10 minutes, and then, 4 mL of a Fehling's solution was added, and the mixture was gently shaken, the mixture was heated in a water bath for 15 minutes, and then cooled to 25°C or lower, then, 2 mL of a potassium iodide solution and 2 mL of sulfuric acid (1 volume of sulfuric acid was diluted with water to make 6 volumes) were added to obtain a test solution. Separately, the same procedure as in the preparation of the test solution was carried out using 10 mL of water instead of the substrate solution to prepare a comparative solution. For the test solution and the comparative solution, the liberated iodine was titrated with a 0.05 mol/L sodium thiosulfate solution. The end point was set to when 1 to 2 drops of a soluble starch test solution were added when the titration was close to the end point, and the resulting blue color disappeared. Under this condition, the amount of enzyme that leads to an increase in reducing power corresponding to 1 mg of glucose per minute was set to 1 unit (1 U), and the glucoamylase activity was calculated from the following formula.

[Mathematical formula 1]

$$\text{Glucoamylase activity (U/g)} = \text{glucose amount (mg)} \times 1/10 \times 1/M$$

$$\text{Glucose amount (mg)} = (b - a) \times 1.6 \times f$$

a: Titration value of test solution (mL)
b: Titration value of comparative solution (mL)
1.6: Here, 1 mL of 0.05 mol/L sodium thiosulfate solution corresponds to the glucose amount of 1.6 mg
1/10: Unit conversion factor of reaction time (min)
M: Amount of enzyme sample in 1 mL of sample solution (g or mL)
f: Factor of 0.05 mol/L sodium thiosulfate solution

<Galactomannanase (GM) activity>

Measurement was performed by the following method based on the ninth edition of the Japan's Specifications and Standards of Food Additives, Hemicellulase Activity Test Method, Method 5.

Here, 0.50 g of an enzyme sample was weighed, and diluted to an appropriate concentration by adding water, and this was used as a sample solution. Then, 0.6 g of locust bean gum (for enzyme) was weighed, and after adding 100 mL of water and stirring, it was heated in a microwave oven 600 W for 2 minutes and then stirred for 1 minute. The mixture was heated again in a microwave oven at 600 W for 2 minutes, stirred for 1 minute, and cooled in flowing water. After cooling, 6 mL of a 5 mol/L hydrochloric acid test solution was added while stirring at room temperature, and after stirring for 15 minutes, 6 mL of a 1 mol/L acetic acid-sodium acetate buffer solution (pH 5.0) was added, and the pH was adjusted to 5.0 using a 0.5 mol/L sodium hydroxide test solution. The volume of the supernatant was adjusted to 300 mL using water and centrifuged at 8000 rpm for 15 minutes to obtain a substrate solution.

In a 50 mL Nessler tube, 4 mL of the substrate solution was weighed, and heated at 40°C for 10 minutes, and then, 1 mL of the sample solution was added, and the mixture was shaken, and heated at 40°C for 10 minutes. Here, 2 mL of a somogyi test solution was added to the solution, and the solution was mixed, and the Nessler tube was slightly capped at the mouth and heated in a water bath for 30 minutes. After cooling, 2 mL of NELSON's test solution was added to this solution, and the mixture was mixed and allowed to stand for 20 minutes, then water was added to make 30 mL, and the mixture was centrifuged at 3000 rpm for 15 minutes, and the supernatant was used as a test solution. Separately, 1 mL of the sample solution was weighed in a 50 mL Nessler tube, 2 mL of the somogyi test solution (I) was added and shaken, then 4 mL of the substrate solution was added and mixed, the mouth of the Nessler tube was lightly stoppered and heated in a water bath for 30 minutes, and the same procedure as in the preparation of the test solution was carried out to prepare a comparative solution. The absorbance of each of the test solution and the comparative solution at a wavelength of 750 nm was measured. Under these conditions, the amount of enzyme that leads to an increase in the reducing power corresponding to 1 μmol of mannose per minute was set to 1 unit (1 U), and the galactomannanase activity was calculated from the following formula.

[Mathematical formula 2]

$$\text{Galactomannanase activity (U/g)} = (A1 - A0) \times 0.163 \times 1/10 \times 1/0.18 \times n$$

A1: Absorbance of Test Solution
A0: Absorbance of Comparative Solution
0.163: Coefficient
1/10: Conversion factor to per minute
1/0.18: Mannose 1 μmol = 0.180 mg
n: Dilution factor per gram of enzyme sample

(2) Experimental methods and results

(2-1) Turbidity and soluble solids content of coffee extract by use of glucoamylase

Here, 150 g of commercially available ground coffee (Columbian Arabica 100%, medium roasted), 150 g of water, and enzymes (Rhizopus oryzae-derived glucoamylase (GA) manufactured by Amano Enzyme Inc., trade name: Gluczyme AF6; hemicellulase manufactured by Amano Enzyme Inc., trade name Hemicellulase "Amano" 90; pectinase manufactured by Amano Enzyme Inc., trade name Pectinase PL "Amano"; pectinase manufactured by Amano Enzyme Inc., trade name Pectinase G "Amano"; Aspergillus niger-derived galactomannanase (GM) manufactured by Amano Enzyme Inc., trade name: Mannanase BGM "Amano" 10; beta-glucanase manufactured by BioCat, trade name Beta-Glucanase 3,000 BGU/g: the same applies to the following:) shown in Table 1 and Table 2 were well mixed in the indicated amounts to prepare a coffee bean slurry, and the coffee bean slurry was put into a French press type coffee maker. Further, 600 g of water was added, and the mixture was allowed to stand at room temperature (25°C) and pH 5.2 for 2 hours. Coffee extract (coffee extraction liquid) was collected by extrusion with a French press.

The turbidity was measured by diluting coffee extract (coffee extraction liquid) with water at Brix = 2 and measuring the turbidity with a turbidity meter (Manufactured by HANNA Corporation, model: HI 93703). The results are shown in Tables 1 and 2.

The soluble solid content yield was determined by measuring Brix and TDS (total dissolved solid content) of a coffee extract (coffee extraction liquid) using a model: PAL-COFFEE (BX/TDS) manufactured by ATAGO CO., LTD., and the soluble solid content yield was calculated based on the following calculation formula. The results are shown in Tables 1 and 2.

[Mathematical formula 3]

$$\text{Soluble solid content yield (\%)} = \text{extraction liquid (mL)} \times \text{TDS (\%)}/\text{coffee beans (g)} \times 100$$

[Table 1]

| | Enzyme used | Use amount (g)/ 1 g coffee beans | Turbidity | | Soluble solid content (%) |
|---|---|---|---|---|---|
| | | | (NTU) | (relative NTU) | |
| Comparative Example 1-1 | None | - | 654 | 1 | 10.4 |
| Example 1-1 | Gluczyme AF6 | 0.001 | 233 | 0.36 | 11.0 |
| Comparative Example 1-2 | Hemicellulase 90 | 0.001 | 475 | 0.73 | 10.7 |
| Comparative Example 1-3 | Pectinase PL | 0.001 | 547 | 0.84 | 10.3 |
| Comparative Example 1-4 | Pectinase G | 0.001 | 480 | 0.73 | 10.6 |
| Comparative Example 1-5 | Mannanase BGM10 | 0.001 | 334 | 0.51 | 10.7 |
| Comparative Example 1-6 | β-glucanase | 0.001 | 505 | 0.77 | 10.8 |

[Table 2]

| | Enzyme used | Use amount | Turbidity | | Soluble solid content (%) |
|---|---|---|---|---|---|
| | | (U/1 g coffee ) | (NTU) | (relative NTU) | |
| Comparative Example 1-1 | None | - | 654 | 1 | 10.4 |
| Example 1-1 | Gluczyme AF6 | 1.6 | 233 | 0.36 | 11.0 |
| Example 1-2 | | 16 | 188 | 0.29 | 11.9 |
| Example 1-3 | | 32 | 125 | | 12.1 |
| Comparative Example 1-5 | Mannanase BGM10 | 10 | 334 | 0.54 | 10.7 |
| Comparative Example 1-7 | | 100 | 335 | 0.51 | 11.3 |
| Comparative Example 1-8 | | 200 | 321 | 0.49 | 11.9 |
| Comparative Example 1-6 | β-glucanase | 3 | 505 | 0.77 | 10.8 |
| Comparative Example 1-9 | | 30 | 549 | 0.84 | 11.4 |
| Comparative Example 1-10 | | 60 | 497 | 0.76 | 11.7 |

As is apparent from Table 1, a remarkable turbidity reducing effect was observed in the case of using glucoamylase (Gluczyme AF6) based on the first embodiment of the present invention (Example 1-1) as compared with the case of using various enzymes considered to be effective in reducing the turbidity of a coffee extract (Comparative Examples 1-2 to 1-6). When glucoamylase (Gluczyme AF6) was used so as to be 8 U per 1 g of coffee beans, Reference Example 2-1 and Reference Example 2-2 (also corresponding to Examples in the first embodiment of the present invention) described later can be referred to. Furthermore, for the case of using glucoamylase (Gluczyme AF6) in combination with galactomannanase (Mannanase BGM 10), the following Examples 2-1 to 2-3 (also corresponding to Examples in the first embodiment of the present invention) can be referred to.

As is apparent from Table 2, when glucoamylase (Gluczyme AF6) was used based on the first embodiment of the present invention, the turbidity reducing effect was improved depending on the amount used (Examples 1-1 to 1-3), whereas when other enzymes were used, the turbidity reducing effect was almost the same regardless of the amount used (Comparative Examples 1-5 to 1-10).

(2-2) Turbidity and soluble solid content of coffee extract by use of glucoamylase and galactomannanase

<Method>

Here, 200 g of commercially available ground coffee (Columbian Arabica 100%, medium roasted), 200 g of water, and the enzyme shown in Table 3 (amount indicated) were well mixed to prepare a coffee bean slurry. The coffee bean slurry was charged into a reactor equipped with a filter paper (No. 1 (pore size = 11 $\mu$m) manufactured by Whatman Corporation) at the bottom, 600 g of water was further added, and the reactor was allowed to stand at room temperature (25°C) and pH 5.2 for 2 hours. From the lower part of the reactor, coffee extract (coffee extraction liquid) was collected by natural fall and extrusion with compressed air.

The turbidity and the soluble solid content yield of the coffee extract (coffee extraction liquid) were measured in the same manner as in the above (3-1). The results are shown in Table 3 below. Fig. 1 shows a graph comparing the turbidity (NTU) in Reference Example 2-1, Examples 2-1 to 2-2, and Comparative Examples 2-2 to 2-3.

[Table 3]

| | Enzyme used | Use amount (U/1 g coffee beans) | Turbidity (NTU) | Turbidity (relative NTU) | Soluble solid content (%) |
|---|---|---|---|---|---|
| Comparative Example 2-1 | None | - | 367 | 1 | 13.9 |
| Reference Example 2-1 | Gluczyme AF6 | 8 | 98 | 0.27 | 14.9 |
| Example 2-1 | Gluczyme AF6 | 6.4 | 108 | 0.29 | 16.4 |
| | Mannanase BGM10 | 10 | | | |
| Example 2-2 | Gluczyme AF6 | 4.8 | 122 | 0.33 | 16.4 |
| | Mannanase BGM10 | 20 | | | |
| Comparative Example 2-2 | Gluczyme AF6 | 4 | 136 | 0.37 | 16.2 |
| | Mannanase BGM10 | 25 | | | |
| Comparative Example 2-3 | Mannanase BGM10 | 50 | 134 | 0.37 | 14.6 |

As is apparent from Table 3, it was found that the yield of soluble solids was significantly improved when enzymes were used in combination (Example 2-1, Example 2-2, and Comparative Example 2-2), as compared with the cases in which glucoamylase (Gluczyme AF6) was used alone (Reference Example 2-1) and galactomannanase (Mannanase BGM 10) was used alone (Comparative Example 2-3).

In addition, as shown in the above (3-1) that glucoamylase (Gluczyme AF6) is extremely excellent in the effect of reducing the turbidity of a coffee extract, the extremely excellent effect is also shown in Table 3 and Reference Example 2-1 in Fig. 1.

However, in the combination of glucoamylase (Gluczyme AF6) and galactomannanase (Mannanase BGM 10) in which the yield of soluble solids is greatly improved, as shown in Fig. 1, when the amount of glucoamylase GA relative to galactomannanase GM was small (Comparative Example 2-2), the effect of reducing the turbidity of a coffee extract by glucoamylase GA was greatly impaired. On the other hand, as shown in Fig. 1, when a predetermined amount or more of glucoamylase GA was used for galactomannanase GM (Examples 2-1 and 2-2, especially Example 2-1), it was possible to suppress the loss of the coffee extract turbidity reducing effect due to the addition of galactomannanase GM. Therefore, by using a predetermined amount or more of glucoamylase GA relative to the galactomannanase GM, an excellent turbidity reduction effect could be obtained while improving the yield of the soluble solid content of the coffee extract.

(2-3) Filtration speed

Here, 200 g of commercially available ground coffee beans, 200 g of water, and enzymes (amounts indicated) shown in Table 4 were well mixed to prepare a coffee bean slurry. The coffee bean slurry was charged into a reactor equipped with a filter paper (No. 1 (pore size = 11 μm) manufactured by Whatman Corporation) at the bottom, 800 g of water was further added, and the reactor was allowed to stand at room temperature for 2 hours. From the lower part of the reactor, coffee extract (coffee extraction liquid) was collected by natural fall and extrusion with compressed air.

The turbidity of the coffee extract (coffee extraction liquid) was measured in the same manner as in the above (3-1). In addition, 200 mL of a coffee extract (coffee extraction liquid) was subjected to suction filtration (-0.5 bar) with filter paper (Manufactured by Whatman, diameter 90 mm, No. 3 (pore diameter = 6 μm)), and the filtration speed (m/h; the amount of filtrate ($m^3$) × 1/filtration area ($m^2$) × 1/hour (hr)) was measured. The results are shown in Table 4. The measurement results of the filtration speed are also shown in Fig. 2.

[Table 4]

|  | Enzyme used | Use amount (U/1 g coffee beans) | Turbidity | | Filtration speed | |
|---|---|---|---|---|---|---|
|  |  |  | (NTU) | (relative NTU) | (m/hr) | (relative m/hr) |
| Comparative Example 2-4 | None | - | 251 | 1 | 0.04 | 1 |
| Reference Example 2-2 | Gluczyme AF6 | 8 | 102 | 0.4 | 0.29 | 8.1 |
| Example 2-3 | Gluczyme AF6 | 6.4 | 157 | 0.62 | 0.24 | 6.7 |
|  | Mannanase BGM10 | 10 |  |  |  |  |
| Comparative Example 2-5 | Mannanase BGM10 | 50 | 170 | 0.68 | 0.16 | 4.5 |

As is clear from Table 4, there was a correlation between turbidity and filtration speed. Specifically, as is apparent from Table 4 and Fig. 2, glucoamylase (Gluczyme AF6) extremely excellent in the effect of reducing the turbidity of a coffee extract also exhibited an extremely high effect on the effect of improving the filtration speed (Reference Example 2-2); while galactomannanase (Mannanase BGM 10) having a poor effect of reducing the turbidity of a coffee extract also has a poor effect of improving the filtration speed (Comparative Example 2-5); in the combination of glucoamylase (Gluczyme AF6) and galactomannanase (Mannanase BGM10) in which the yield of soluble solid content is greatly improved, when a predetermined amount of glucoamylase (GA) was used for galactomannanase (GM) (Example 2-3), an excellent overspeed-increasing effect was obtained. Therefore, by using a predetermined amount of glucoamylase (GA) relative to galactomannanase (GM), it was possible to obtain an excellent turbidity reduction effect and an excellent filtration speed improvement effect while improving the yield of the soluble solid content of the coffee extract.

(2-4) Turbidity change of coffee extract by use of glucoamylase and galactomannanase

<Method>

Here, 150 g of commercially available ground coffee (Columbian Arabica 100%, medium roasted), 150 g of water, Gluczyme AF6 (6.4 U/1 g of coffee beans) and Mannanase BGM10 (10 U/1 g of coffee beans) were well mixed to form a coffee bean slurry, and the coffee bean slurry was charged into a French press type coffee maker. Further, 450 g of water was added, and the mixture was allowed to stand at room temperature (25°C) for 2 hours. Coffee extract (coffee extraction liquid) was collected by extrusion with a French press. Subsequently, the recovered extract was concentrated to about 40 mL by vacuum concentration (60°C, 30 rpm, -1 bar) to obtain a coffee concentrate. Next, to this concentrated solution, diatomaceous earth (Celite Hyflo) was added in a liquid amount of 3%, and suction filtration (-1 bar) was performed with filter paper coated with diatomaceous earth (Celite Hyflo), and heat treatment (90°C, 5 min) was further performed to obtain a clarified coffee concentrate. The obtained concentrated solution was stored at 4°C. In order to confirm the change in turbidity over time during storage, the solution was diluted with water so that Brix = 2, and the turbidity was measured with a turbidity meter (Manufactured by HANNA Corporation, model: HI 93703). The turbidity (NTU) immediately after the preparation of Comparative Example 2-6 was set to 1, and the relative turbidity at each time point was derived. The results are shown in Table 5 below.

[Table 5]

| | Enzyme used | Used amount (U/1 g coffee beans) | Turbidity (relative NTU) | | | |
|---|---|---|---|---|---|---|
| | | | Immediately after preparation | After 1 month | After 2 months | After 3 months |
| Comparative Example 2-6 | None | - | 1 | 12.2 | 17.0 | 16.8 |
| Example 2-4 | Gluczyme AF6 | 6.4 | 0.67 | 1.1 | 1.2 | 1.3 |
| | Mannanase BGM10 | 10 | | | | |

As is apparent from Table 5, when glucoamylase (Gluczyme AF6) and galactomannanase (Mannanase BGM 10) were used in combination (Example 2-4), not only the turbidity immediately after the preparation of the coffee extract was remarkably suppressed, but also the increase in turbidity during storage of the coffee extract was remarkably suppressed as compared with the case where no enzyme was used (Comparative Example 2-6), so that it could be confirmed that more remarkable stability (turbidity reducing effect) was achieved after storage of the coffee extract.

**Claims**

1.   A method for producing a coffee extract, the method comprising a step of bringing a coffee extract into contact with glucoamylase having a glucoamylase activity of 32 U or less per 1 g of coffee beans.

2.   The method according to claim 1, wherein in the step, a coffee extract is extracted from a slurry containing ground coffee beans, water, and the glucoamylase.

3.   The method according to claim 1 or 2, wherein the glucoamylase is used at a glucoamylase activity of 0.5 U or more per 1 g of the coffee beans.

4.   A method for producing a coffee extract, the method comprising a step of bringing a coffee extract into contact with glucoamylase and galactomannanase, wherein the glucoamylase is used at a glucoamylase activity of 0.24 U or more per 1 U of the galactomannanase activity.

5.   The method according to claim 4, wherein in the step, a coffee extract is extracted from a slurry containing ground coffee beans, water, the glucoamylase, and the galactomannanase.

6.   The method according to claim 4 or 5, wherein the glucoamylase is used at a glucoamylase activity of 2 U or less per 1 U of the galactomannanase activity.

7.   The method according to any one of claims 1 to 6, wherein the glucoamylase is used at a glucoamylase activity of 20 U or less per 1 g of coffee beans.

8.   The method according to any one of claims 1 to 7, wherein the glucoamylase is derived from Rhizopus oryzae.

9.   An enzyme preparation comprising glucoamylase for the use of reducing turbidity of a coffee extract.

10.   The enzyme preparation according to claim 9, for the use in an amount that the glucoamylase activity is 32 U or less per 1 g of coffee beans.

11.   The enzyme preparation according to claim 9 or 10, further comprising galactomannanase, wherein a content of the glucoamylase per 1 U of the galactomannanase activity is 0.24 U of glucoamylase activity.

FIG. 1

FIG. 2

## INTERNATIONAL SEARCH REPORT

| | International application No. |
|---|---|
| | **PCT/JP2021/031574** |

**A. CLASSIFICATION OF SUBJECT MATTER**

*A23F 5/24*(2006.01)i
FI:  A23F5/24

According to International Patent Classification (IPC) or to both national classification and IPC

**B. FIELDS SEARCHED**

Minimum documentation searched (classification system followed by classification symbols)

A23F5/24

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2009-278957 A (HASEGAWA T. CO., LTD.) 03 December 2009 (2009-12-03) claims, paragraphs [0001], [0004], [0024], [0026], [0027] | 1-11 |
| A | JP 2003-299441 A (UCC UESHIMA COFFEE CO., LTD.) 21 October 2003 (2003-10-21) claims 1, 2, paragraphs [0021]-[0023] | 1-11 |
| A | JP 7-184546 A (KIRIN BEVERAGE COMPANY LTD.) 25 July 1995 (1995-07-25) claim 1, paragraph [0001] | 1-11 |
| A | 岩井和也. 食品酵素の最新利用技術　ガラクトマンナナーゼを用いたコーヒー製品の品質改善. BIO INDUSTRY. 12 October 2006, vol. 23, no. 10, pp. 32-39, (IWAI, Kazuya, FUKUNAGA, Taiji. Quality Improvement of Coffee Products by Using Galactomannanase), non-official translation (Latest Uses of Enzyme Foods.) p. 32, "2. Enzyme Uses", p. 38, "8. Conclusion" | 1-11 |

☐ Further documents are listed in the continuation of Box C.       ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 October 2021** | **19 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2021/031574**

| Patent document cited in search report | | | Publication date (day/month/year) | Patent family member(s) | Publication date (day/month/year) |
|---|---|---|---|---|---|
| JP | 2009-278957 | A | 03 December 2009 | (Family: none) | |
| JP | 2003-299441 | A | 21 October 2003 | (Family: none) | |
| JP | 7-184546 | A | 25 July 1995 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H04045745 A
- JP 2002272375 A
- JP 2002330700 A
- JP 2003047406 A